# EUROPEAN PATENT APPLICATION

(11) **EP 4 481 034 A1**
(43) Date of publication of application: **25.12.2024**
(21) Application number: 23756377.0
(22) Date of filing: 15.02.2023
(51) Int. Cl.: C12N 5/0735, C12N 5/074, C12N 5/10, A61L 27/36

(54) **METHOD FOR INDUCING DIFFERENTIATION FROM PLURIPOTENT STEM CELL TO EPIDERMAL KERATINOCYTE**

(30) Priority: 16.02.2022 JP 2022022101
(71) Applicant: Kose Corporation, Tokyo 103-8251 (JP); The Jikei University, Tokyo 105-8461 (JP)
(72) Inventor: OBAYASHI NARITA, Junko, Tokyo 114-0005 (JP); WASHIZU, Kaho, Tokyo 114-0005 (JP); NARU, Eiji, Tokyo 114-0005 (JP); ITOH, Munenari, Tokyo 105-8461 (JP); KAWAGOE, Shiho, Tokyo 105-8461 (JP)
(74) Representative: D Young & Co LLP
(86) International application number: PCT/JP2023/005094
(87) International publication number: WO 2023/157852

(57) **Abstract**

[Summary]

[Problems] The main purpose of this invention is to provide a technique for inducing an epidermal keratinocyte that can produce a three-dimensional cultured skin model with a multi-layered horny cell layer, from a pluripotent stem cell.

[Solution] The present invention is a method for inducing differentiation from a pluripotent stem cell to an epidermal keratinocyte, using a non-detached cell after being treated with a cell detaching agent in the inducing step from the pluripotent stem cell, wherein when applying the epidermal keratinocyte to a three-dimensional cultured skin model, a three-dimensional cultured skin model in which the horny cell layer is multi-layered is obtained.

## Description

### Technical field

The present invention relates to a method for inducing differentiation from a pluripotent stem cell to an epidermal keratinocyte, a method for producing an epidermal keratinocyte, a method for producing a three-dimensional cultured skin model, and the like.

### Background Art

Pluripotent stem cells are attracting attention in a wide range of fields, including the medical and cosmetic fields and the like, such as transplant therapy, regenerative medicine, cosmetics, drug discovery, custom-made products for symptoms and diseases (medicines, medical care), custom-made products for the skin (cosmetics, skin care, etc.), and the like, and are being researched, developed, and further, utilized in these fields, as stem cells that can differentiate into almost all cells that make up the body. Then, as pluripotent stem cells that have been established to date, somatic stem cells and the like are known such as ES cells (embryonic stem cells), EG cells (embryonic germ cells), iPS cells (also referred to as "artificial pluripotent stem cells"), mesenchymal stem cells (MSC), Muse cells (multi lineage differentiating stress enduring cells), and the like, and the number of types of the pluripotent stem cells will likely increase with future technological developments and new knowledge.

For example, ES cells are known as cells created from a part of an embryo at the blastocyst stage after repeated division of a fertilized egg of a mammal such as a human or the like. In addition, iPS cells are pluripotent stem cells that are artificially created by culturing cells, and specifically, known as cells that transform into pluripotent stem cells that have the ability to differentiate into cells of various tissues and organs and the ability to proliferate almost indefinitely, by introducing a very small number of factors (undifferentiated cell-specific genes) into somatic cells such as the skin or blood of mammals such as humans and culturing them. In addition, with the recent development of direct reprogramming technology, mesenchymal stem cells and Muse cells have come to be used as somatic stem cells, and for example, Muse cells exist in the blood, bone marrow, and connective tissues of various organs, and are known to have the ability to differentiate into various cells of endoderm (lungs, liver, pancreas, etc.), mesoderm (heart, kidneys, bones, blood vessels, etc.), and ectoderm (neural tissue, epidermis, etc.).

It is also known that a pluripotent stem cell, such as embryonic stem cells and artificial pluripotent stem cells, can be grown for long periods in vitro while retaining the ability to differentiate to all types of cells in the body, including keratinocytes, and various studies have been conducted on inducing differentiation of these stem cells to keratinocytes.

For example, Patent Literature 1 discloses a method for providing engraftable keratinocyte stem cells by differentiation of a pluripotent stem cell, which includes (a) forming the aggregate of a pluripotent stem cell in suspension culture in the presence of a defined basal medium; (b) culturing the aggregate in suspension culture in the presence of an initiation medium containing retinoic acid and BMP4 to promote the formation of the initiation aggregate; (c) culturing the initiation aggregate in a keratinocyte precursor medium containing cholera toxin and a TGFβR1 kinase inhibitor to promote the formation of a cell population containing keratinocyte precursor cells; and (d) culturing keratinocyte precursor cells in a keratinocyte stem cell maturation medium to promote the formation of a cell population containing engraftable keratinocyte stem cells.

For example, Patent Literature 2 discloses an ex vivo method for obtaining a human keratinocyte population derived from human pluripotent stem cells, including a step of co-culturing human pluripotent stem cells with cells supporting ectodermal differentiation in the presence of an agent that stimulates epidermal induction and an agent that stimulates terminal differentiation of keratinocytes.

For example, Patent Literature 3 discloses a method for preparing an organotypic vascularized tissue, skin, or mucosal equivalent or composition, including (i) a step of inducing formation of the following differentiated cell types: endothelial cells (SC-EC), vascular smooth muscle cells/pericytes (SC-vSMC), fibroblasts (SC-Fib), and keratinocytes (SC-KC), by obtaining a preparation of mammalian pluripotent stem cells and culturing the cells under cell culture conditions, (ii) a step of inducing formation of a vascularized dermal layer, by seeding the SC-EC, SC-vSMC, and optionally SC-Fib in the part (i) into or on a scaffold and further culturing the cells under cell culture conditions, and (iii) a step of inducing the formation of a multi-layered layer of keratinized epidermis on the vascularized dermal layer, by seeding the SC-KC of the part (i) on the vascularized dermal layer of the part (ii) and further culturing the cells under cell culture conditions, thereby obtaining an organotypic vascularized skin or mucosal equivalent.

### Citation List

### Patent Literature

Patent Literature 1: Japanese Unexamined Patent Application Publication (Translation of PCT Application) No. 2017-532047
Patent Literature 2: Japanese Unexamined Patent Application Publication (Translation of PCT Application) No. 2011-525370
Patent Literature 3: Japanese Unexamined Patent Application Publication (Translation of PCT Application) No. 2018-518970

### Summary of Invention

### Technical Problem

The present inventors decided to consider obtaining epidermal keratinocytes using pluripotent stem cells and creating a three-dimensional cultured skin model using the epidermal keratinocytes, since stem cells have pluripotency and can be proliferated for a long period of time. The present inventors thought that experiments related to skin physiological function research including gene editing experiments, which could not be conducted in experimental systems using conventional cells, could be conducted more easily, by establishing a method for creating a three-dimensional cultured skin model from pluripotent stem cells.

However, the present inventors obtained epidermal keratinocytes from iPS cells, which are pluripotent stem cells, using a method for inducing differentiation to epidermal keratinocytes that has already been reported in literatures and patent documents, and created a three-dimensional cultured skin model using the iPS cell-derived epidermal keratinocytes, and confirmed and studied the condition of the skin model, and as a result, the skin model did not have a horny cell layer, or the horny cell layer was so thin that a continuous multi-layered portion was not formed over a plane area larger than a predetermined area. The present inventors found a problem that a three-dimensional cultured skin model in which the horny cell layer has a thickness greater than a predetermined value but is not continuously multi-layered over an area size is likely to cause variations in test results, and therefore, it cannot be used for skin physiological function research and the like. As a result of extensive investigation, the present inventors found that the cause of this is that in the conventional differentiation induction method for converting a pluripotent stem cell to an epidermal keratinocyte, the quality or quantity of cells obtained after differentiation induction (for example, the quality or quantity of an epidermal keratinocyte derived from a pluripotent stem cell in the obtained cell group) has a problem.

Therefore, the main object of the present invention is to provide a technique for inducing an epidermal keratinocyte, which can produce a three-dimensional cultured skin model having a multi-layered horny cell layer, from a pluripotent stem cell.

### Solution to Problem

As a result of extensive research, the present inventors found that in inducing differentiation from a pluripotent stem cell to an epidermal keratinocyte, if culturing is performed at least once using cells that could not be detached from cell culture equipment with a cell detaching agent (hereinafter, also referred to as "non-detached cell") to obtain epidermal keratinocytes and the epidermal keratinocytes are applied to a three-dimensional cultured skin model in the middle of the process, then, a three-dimensional cultured skin model in which the horny cell layer is multi-layered is obtained.

Normally, detached cells that have been detached with a cell detaching agent from cell culture equipment are sorted and collected and culturing is performed using the collected detached cells, and therefore, non-detached cells that remained on the cell culture equipment because they could not be detached with the cell detaching agent were disposed of along with the cell culture equipment. However, the present inventors have newly discovered that in the differentiation induction process, if these non-detached cells are deliberately cultured ongoingly until the cells become confluent to obtain a cell group and the epidermal keratinocytes obtained from this cell group are applied to a three-dimensional cultured skin model, then, a three-dimensional cultured skin model in which the horny cell layer is continuously multi-layered is obtained. As a result, the present inventors have provided a method for inducing differentiation from a pluripotent stem cell to an epidermal keratinocyte, a method for producing an epidermal keratinocyte, a method for producing a three-dimensional cultured skin model, and the like.

The present invention can provide a method for inducing differentiation from a pluripotent stem cell to an epidermal keratinocyte, using a non-detached cell after being treated with a cell detaching agent in the inducing step from the pluripotent stem cell,
wherein when applying the epidermal keratinocyte to a three-dimensional cultured skin model, a three-dimensional cultured skin model in which the horny cell layer is multi-layered is obtained.

The present invention can provide a method for producing an epidermal keratinocyte, which includes a step of inducing differentiation from a pluripotent stem cell to an epidermal keratinocyte, using a non-detached cell after being treated with a cell detaching agent.

The present invention can provide a method for producing a three-dimensional cultured skin model using an epidermal keratinocyte produced using the method for inducing differentiation from a pluripotent stem cell to an epidermal keratinocyte.

### Advantageous Effects of Invention

The present invention can provide a technique for inducing an epidermal keratinocyte, which can produce a three-dimensional cultured skin model having a multi-layered horny cell layer, from a pluripotent stem cell.

### Brief Description of Drawing

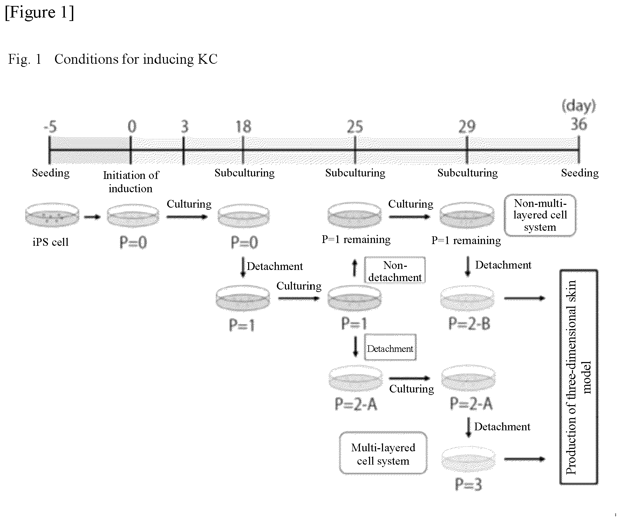

[Figure 1] Figure 1 shows a schematic view of the study of conditions for inducing differentiation from a pluripotent stem cell to an epidermal keratinocyte. Note that KC is an abbreviation for keratinocyte (epidermal keratinocyte).

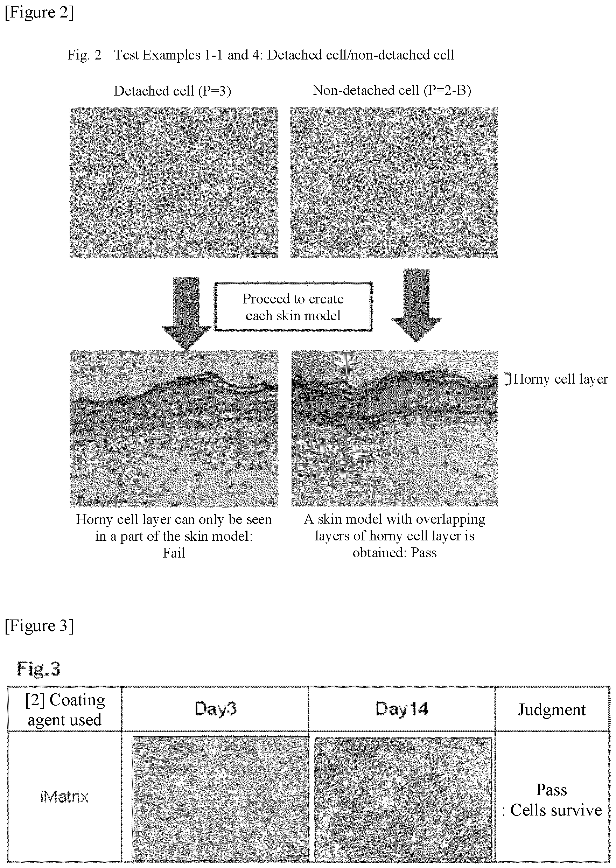

[Figure 2] The left figure is a view when producing a three-dimensional cultured skin model using detached cell line epidermal keratinocytes. The right figure is a view when producing a three-dimensional cultured skin model using non-detached cell line epidermal keratinocytes. When non-detached cell line epidermal keratinocytes are used, a three-dimensional cultured skin model having a multi-layered horny cell layer can be produced.
[Figure 3] FIG. 3 is a microscopic observation view showing the state of cells when iMatrix coating agent (culture substrate: laminin 511-E8 fragment) is used for cell culture equipment.

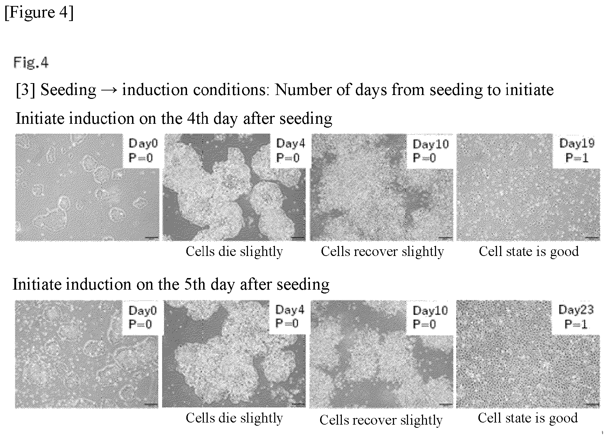

[Figure 4] FIG. 4 is a view showing the state of cells observed by optical microscopy in the induction step after the initiation of induction in the cases where the number of days from seeding to the initiation of culture in the pluripotent stem cell maintenance step is 4 days and 5 days.

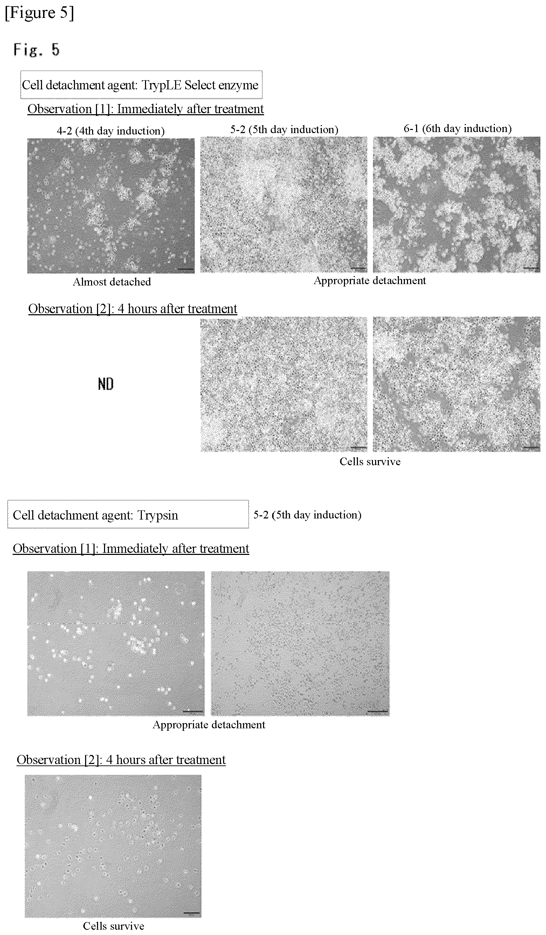

[Figure 5] FIG. 5 is a view showing the detachment state when using TrypLE (trademark) Select and trypsin cell detaching agents. The upper part of Figure 5 "Cell detaching agent: TrypLE (trademark) Select enzyme" shows the results of TrypLE (trademark) Select enzyme, and this shows the cell condition in the cases where the number of days from seeding to the initiation of culture in the pluripotent stem cell maintenance step is 4 days, 5 days and 6 days. The lower part of FIG. 5 "Cell detaching agent: Trypsin" is a view showing the results of trypsin, and this view shows the cell condition in the case where the number of days until the initiation of culture is 5 days.

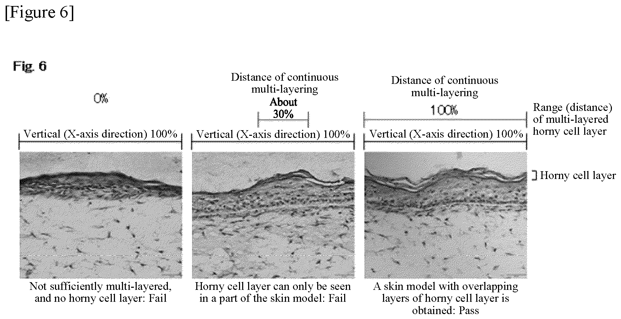

[Figure 6] Figure 6 is an example view showing the pass/fail evaluation criterion for the three-dimensional cultured skin model in the present embodiment, and shows cross sections in which the three-dimensional cultured skin model is cut in the vertical direction and observed under an optical microscope after being stained with hematoxylin and eosin (HE). The left figure is an example view of a failure (×) in which no horny cell layer is formed and the range of the multi-layered horny cell layer (X-axis direction) is 0%. The center view is an example view of a failure (×) in which multi-layering of the horny cell layer is seen only within the specified range (X-axis direction), and the range of the multi-layered horny cell layer (X-axis direction) is 30% whereas the pass criterion is 80% or higher. The right figure is an example view of a pass (∘) in which multi-layering of the horny cell layer is seen over the specified range (X-axis direction), and the range of the multi-layered horny cell layer (X-axis direction) is 100% whereas the pass criterion is 80% or higher.

### Description of Embodiments

A preferred embodiment for carrying out the present technique will be described below. The embodiment described below shows an example of a typical embodiment of the present technique, and the scope of the present technique will not be narrowly interpreted by this. In addition, percentages in the present specification are expressed by mass (mass/mass%) unless otherwise specified. In addition, the upper limit (or less) and the lower limit (or more) of each numerical range (-) can be arbitrarily combined as desired.

### 1. Method for inducing differentiation from pluripotent stem cell to epidermal keratinocyte according to the present embodiment

The embodiment in the present invention provides a method for inducing differentiation from a pluripotent stem cell to an epidermal keratinocyte, using a non-detached cell after being treated with a cell detaching agent in the inducing step from the pluripotent stem cell, and is preferably characterized in that when an epidermal keratinocyte is applied to a three-dimensional cultured skin model, a three-dimensional cultured skin model in which the horny cell layer is multi-layered is obtained. Furthermore, the present embodiment may also be a method for inducing differentiation from a pluripotent stem cell to an epidermal keratinocyte, for obtaining a three-dimensional cultured skin model in which the horny cell layer is multi-layered when the epidermal keratinocyte is applied to a three-dimensional cultured skin model.

Moreover, the present embodiment may be a method for inducing differentiation from a pluripotent stem cell to an epidermal keratinocyte, at least including inducing differentiation from a pluripotent stem cell to an epidermal keratinocyte. The method for inducing differentiation from a pluripotent stem cell to an epidermal keratinocyte preferably includes at least culturing a pluripotent stem cell maintained in an undifferentiated state or culturing after a subculture operation, and inducing differentiation from the pluripotent stem cell maintained in an undifferentiated state to an epidermal keratinocyte. Moreover, the differentiation induction method in the present embodiment may also be a method for producing an epidermal keratinocyte from a pluripotent stem cell.

In addition, in the present specification, "subculture operation" means an operation of collecting a part of cultured cells and transferring them to a new separate culture container, and "culture" refers to, though not particularly limited to, for example, seeding cells on a culture scaffold and maintaining or division-proliferating, or differentiating them.

In the present specification, "--- ing" of "inducing differentiation" and the like may be replaced with "process" or "step", "process" may be replaced with "--- ing" or "step", and "step" may be replaced with "--- ing" or "process". Furthermore, in the present embodiment, "process" of "process" and the like may be "a device or a unit configured to perform a process," and "device" may be "a mechanism or a unit", and alternatively, "unit" may be "a unit or device to be provided in a mechanism, device, or system".

Further, it is preferable that the present embodiment includes at least a step of inducing differentiation from a pluripotent stem cell to an epidermal keratinocyte. Furthermore, it may provide a method for producing an epidermal keratinocyte, including at least an undifferentiation maintenance step of culturing or subculturing a pluripotent stem cell in an undifferentiated state, and a step of inducing differentiation from the pluripotent stem cell that have undergone the undifferentiation maintenance step to an epidermal keratinocyte, or may provide an epidermal keratinocyte obtained by the production method.

Furthermore, another embodiment of the present invention may provide a method for producing an epidermal keratinocyte, including a step of inducing differentiation from a pluripotent stem cell to an epidermal keratinocyte using a non-detached cell after being treated with a cell detaching agent.

Further, still another embodiment of the present invention may provide an epidermal keratinocyte produced using the method for inducing differentiation from a pluripotent stem cell to an epidermal keratinocyte, or may provide a method for producing the epidermal keratinocyte.

Furthermore, the present embodiment may provide a method for producing a three-dimensional cultured skin model using an epidermal keratinocyte obtained using the differentiation induction method according to the present embodiment. In addition, the present embodiment may provide a method for producing a three-dimensional cultured skin model, including a step of inducing differentiation from a pluripotent stem cell to an epidermal keratinocyte, and a step of constructing a three-dimensional cultured skin model using an epidermal keratinocyte derived from a pluripotent stem cell after the step of inducing differentiation.

### 1-1. Raw materials used, etc.

### 1-1-1. Pluripotent stem cell

In the present embodiment, a pluripotent stem cell is used.

The pluripotent stem cell used in the present invention is a stem cell that has pluripotency that can be differentiated to all cells existing in a living body and also have the ability to proliferate, and examples thereof include, but are not limited to, embryonic stem cells (ES cells), embryonic stem cells (ntES cells) derived from cloned embryos by nuclear transfer, sperm stem cells (GS cells), embryonic germ cells (EG cells), artificial pluripotent stem cells (iPS cells), cultured fibroblast and bone marrow stem cell-derived somatic stem cells (Muse cells, mesenchymal stem cells, etc.), and the like, and one or two or more selected from these can be used. These cells can be produced by known production methods or obtained from the market, public institutions, etc. The cells are preferably derived from mammalian cells, particularly human cells.

Embryonic stem cells (ES cells) are stem cells established from the inner cell mass of early embryos (e.g., blastocysts) of mammals such as humans and mice. Sperm stem cells are pluripotent stem cells derived from sperm and are the originating cells for spermatogenesis. Embryonic germ cells are cells established from embryonic primordial germ cells.

Artificial pluripotent stem cell (iPS cells) are artificial stem cells derived from somatic cells that have properties almost equivalent to ES cells, such as pluripotency and the ability to proliferate through self-renewal, which can be created by introducing certain reprogramming factors in the form of DNA or proteins into somatic cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); International Publication WO2007/069666). The reprogramming factor may be a gene that is specifically expressed in ES cells, a gene that plays an important role in maintaining the undifferentiated state of ES cells, or its gene product, and is not particularly limited, but includes, for example, combinations of OCT3/4, SOX2 and KLF4; OCT3/4, KLF4 and C-MYC; OCT3/4, SOX2, KLF4 and C-MYC; OCT3/4 and SOX2; OCT3/4, SOX2 and NANOG; OCT3/4, SOX2 and LIN28; OCT3/4 and KLF4; and the like.

The pluripotent stem cells are preferably ES cells and/or iPS cells, more preferably iPS cells. The iPS cells are preferably derived from mammalian (preferably human) cells. Generally, cells used for producing iPS cells are not particularly limited, and iPS cells derived from somatic cells are preferred.

In the present embodiment, the somatic cells used for producing iPS cells are preferably differentiated cells, and among the differentiated cells, skin-based cells are preferable, and among these, keratinocytes (also referred to as "epidermal keratinocyte", "skin keratinocytes" or "keratinocytes") are more preferred. Furthermore, in the present embodiment, iPS cells produced using keratinocytes (hereinafter also referred to as "keratinocyte-derived iPS cells") are preferable from the standpoint of obtaining an epidermal keratinocyte with which a three-dimensional cultured skin model in which the horny cell layer is multi-layered can be produced more successfully and more efficiently. Note that iPS cells derived from an epidermal keratinocyte can be obtained using a known method for producing iPS cells derived from an epidermal keratinocyte (for example, Reference 1: Okita K. et. al., Nature Methods, 2011,8:409-12.).

Note that the somatic cells used in the present embodiment refer to all animal cells (preferably animal cells of mammals including humans) excluding germline cells such as eggs, oocytes, and ES cells, or totipotent cells. Somatic cells include, without limitation, any of fetal (child) somatic cells, newborn (child) somatic cells, and mature healthy or diseased somatic cells, and include any of primary cultured cells, subcultures and established cell lines.

More specific examples of somatic cells include tissue stem cells (somatic stem cells) such as neural stem cells, hematopoietic stem cells, mesenchymal stem cells, dental pulp stem cells, and the like; tissue progenitor cells; differentiated cells such as lymphocytes, epithelial cells, endothelial cells, muscle cells, fibroblasts, keratinocytes, hair cells, stem cells, gastric mucosal cells, intestinal cells, splenocytes, pancreatic cells, brain cells, embryonic cells, kidney cells, adipocytes, and the like; etc., and one or two or more selected from these can be used.

### 1-1-2. Culture substrate (coating agent)

In the present embodiment, it is preferable to use a coating agent containing a culture substrate, and use the coating agent to coat the area where the cell culture equipment and cells are in contact with each other with the culture substrate. By coating the surface of the cell culture equipment with the culture substrate, the adhesion of cells to the surface of the cell culture equipment can be enhanced or improved. Examples of the coating treatment include placing a solution containing a culture substrate into the cell culture equipment and then removing the solution as appropriate, but the method is not particularly limited thereto, and any known coating treatment may be employed as appropriate.

Examples of culture substrates include, but are not limited to, extracellular matrices such as laminin, collagen, fibronectin, vitronectin, matrigel, fibrin, and thrombin; amino acid polymers such as poly-L-lysine and poly-D-lysine; and fragments thereof, and one or two or more selected from these can be used.

Among the culture substrates, laminin and its fragments are preferred.

As laminin and its fragments, it is preferable to use laminin 511 (laminin consisting of α5 chain, β1 chain, and γ1 chain) and fragments thereof. Laminin is a major cell adhesion molecule in the basement membrane, and is a heterotrimer consisting of three subunit chains: α chain, β chain, and γ chain, and is a huge glycoprotein with a molecular weight of approximately 800,000 Da. It is a heterotrimeric molecule in which three subunit chains come together at the C-terminus to form a coiled-coil structure and are stabilized by disulfide bonds. Therefore, laminin 511 means laminin in which the α chain is α5, the β chain is β1, and the γ chain is γ1.

Furthermore, laminin may be a mutant, and is not particularly limited as long as it has integrin binding activity. Laminin of human origin is preferred. Laminin and its fragments are preferably those that exhibit a binding activity with integrin α6B1 with a dissociation constant of 10 nM or less. It is preferable to use commercially available laminin or laminin fragments.

As a laminin fragment, E8 fragment (also referred to as laminin 511E8 fragment or laminin 511E8) which is a fragment obtained by digesting laminin 511 with elastase (Reference 2: Ido H, et al, J Biol Chem. 2007, 282, 11144 -11154), recombinant human laminin 511E8 fragment expressed from gene recombinant silkworm cocoons, and the like, are listed.

Among laminin and its fragments, laminin fragments are preferred, more preferably laminin 511 fragments, even more preferably laminin 511E8 fragments, and human-derived fragments are more preferred.

The cell culture equipment to be coated with the culture substrate (coating agent) is usually suitably cell culture equipment that can be used for cell culture of a pluripotent stem cell, and examples thereof include, but not limited to, flasks, dishes, petri dishes, bottles, plates, etc. The material (made) of the cell culture equipment is not particularly limited, but one or two or more selected from synthetic resins (preferably plastics) such as styrene resins (polystyrene or styrene copolymer, etc.), polycarbonates, polyolefin resins (polyethylene, polypropylene, polyester, ethylene copolymer, etc.), (meth)acrylic resins, silicone resins, amino resins, fluororesins, polyimide resins, and the like; and glass base materials, are suitable. Among these, plastics (more preferably styrene resins, polyolefin resins (polyethylene, polypropylene), styrene resins, polycarbonates) are preferred, and among these, styrene resins (more preferably polystyrene) are more preferred.

### 1-1-3. Cell detaching agent

In the present embodiment, a cell detaching agent is used, and the cell detaching agent is preferably a cell detachment component that is generally used to detach cell culture equipment from cultured cells during animal cell culture, or an agent containing the component.

Components used for cell detachment include serine proteases such as trypsin proteases (trypsin, trypsin-like proteases, etc.) and chymotrypsin; metalloproteases such as dispase; Ca-dependent proteases such as actinase; neutral protease; enzyme stripping systems such as protease having protease and collagenase activity, enzyme-free stripping systems such as ethylenediaminetetraacetic acid (EDTA) and sodium citrate; TrypLE (trademark); and the like, and one or two or more selected from these can be used, but are not particularly limited to these. As the cell detaching agent, a commercially available cell detaching agent may be used. Further, as the cell detachment means, a physical cell detachment means may be used, and for example, a cultured cell detachment device such as Cell Scraper (produced by Corning Inc.) may be used.

Among the cell detaching agents, enzymes or those containing enzymes are preferable, and as the enzymes, protease enzymes are more preferable, trypsin enzymes are even more preferable, and trypsin-based protease such as trypsin and trypsin-like proteases, TrypLE (trademark) select enzyme, and the like; TrypLE (trademark) select enzyme; recombinant protein having trypsin-like activity (more preferably trypsin-like protease derived from a gene recombinant microorganism) are more preferable. The term "trypsin-based enzyme" includes trypsin and trypsin-like enzymes.

"Trypsin-like" is preferably one that exhibits the same or similar kinetics and cutting properties as trypsin, and more preferably can directly replace trypsin in cell detachment without changing the protocol. The cutting property of trypsin is that it can hydrolyze peptide bonds on the carboxy group side of basic amino acids (lysine, arginine).

In addition, the enzyme may be derived from either animals or microorganisms, but it is preferably derived from microorganisms, and more preferably from a gene recombinant microorganism that has been gene recombinant to be able to produce protease or trypsin, and furthermore, those derived from gene recombinant fungi are even more preferable.

The cell detaching agent preferably contains protease and/or TrypLE (trademark) select enzyme, and as the protease, one or two or more selected from trypsin, trypsin-like protease, and TrypLE (trademark) select enzyme are more preferably used, with trypsin and/or TrypLE (trademark) select enzyme being further preferable. Suitable cell detaching agents include, for example, trypsin, TrypLE (trademark) select enzyme, and cell detaching agents containing the TrypLE (trademark) select enzyme and 0.5 to 1.5 mM EDTA; and the like, and one or two or more selected from them can be used.

Note that it is preferable to use a physical treatment in combination with the treatment of detaching adherent cells from the cell culture equipment using a cell detaching agent. The physical treatments include, for example, water flow generation means such as pipetting operations (suction, spraying, etc.), scraping means such as a spatula-shaped scraper capable of detaching adherent cells, etc.; and water flow generating means (preferably collection of detached cells after detachment by suction and spraying) is suitable from the viewpoint of reducing cell damage.

Further, the collected detached cells may be subjected to separation means such as centrifugation, whereby single cells may be further collected and used for the next cell culture.

### 1-1-4. Culture medium for pluripotent stem cell

As a culture medium for a pluripotent stem cell, a known culture medium generally used for culturing a pluripotent stem cell or a commercially available culture medium can be used. Examples of culture media for a pluripotent stem cell include culture media containing one or two or more selected from serum-containing or serum-free media, on-feeder or feeder-free media, and the like.

Note that the term "serum-free medium" or "medium containing no serum" refers to a medium that does not contain untreated or unpurified serum; for example, a medium that contains purified blood-derived components or animal tissue-derived components (e.g., growth factors) may be included. As the serum, serum obtained from the same animal as the stem cells is preferred from the viewpoint of preventing contamination with components derived from a different species of animal.

In addition, feeder cells refer to other cell types that play an auxiliary role and are used to adjust the culture conditions for target cells whose proliferation and differentiation are to occur. Usually, feeder cells are treated in advance with gamma ray irradiation or antibiotics to prevent them from proliferating.

The culture medium for a pluripotent stem cell is preferably an animal-derived component-free (Xeno-free) and/or feeder-less (or feeder free) medium, more preferably an animal-derived component and feeder free medium, and further, it is more preferable to use an undifferentiation maintenance medium that achieves undifferentiation maintenance culture in which human pluripotent stem cells (ES cells, iPS cells) are grown while maintaining their undifferentiated state, that is also suitable for maintaining the ability to differentiate into keratinocytes, and that can be used for undifferentiation maintenance culture. The culture medium for a pluripotent stem cell may also contain serum substitutes, such as albumin, vegetable starch, dextran, fatty acids, collagen, etc., and one or two or more selected from these can be used. Furthermore, it can contain one or two or more selected from fatty acids, fats, amino acids, vitamins, growth factors, cytokines, antioxidants, buffers, inorganic salts, and the like.

Commercially available culture media for a pluripotent stem cell include, for example, StemFit (R) AK02N; commercially available basal media include, for example, TeSR, Essential 8 medium, BME, BGJb, CMRL1066, Glasgow MEM, and improved MEM zinc option (Zinc Option), IMDM, Medium 199, Eagle MEM, αMEM, DMEM, Ham, RPMI 1640, Fisher's medium, etc., and one or two or more selected from these can be used, but the medium is not limited to these. Among these, StemFit (R) AK02N is suitable as a medium for maintaining undifferentiated pluripotent stem cell, and as a medium for differentiation from a pluripotent stem cell to an epidermal keratinocyte (differentiation induction initiation medium and/or differentiation maintenance medium), CnT-07 medium is suitable, and these media are more suitable for conducting culturing using iPS cells derived from human epidermal keratinocyte (also referred to as iPS cells derived from human epidermal keratinocyte or human keratinocytes).

By blending components or agents for various purposes such as pluripotent stem cell undifferentiation maintenance and differentiation induction, suppressing apoptosis, and improving survival rate to the culture medium of a pluripotent stem cell, it is possible to perform pluripotent stem cell undifferentiation maintenance and differentiation induction. As ingredients or agents for various purposes to be blended, commercially available products can be used as appropriate.

For example, a commonly used culture medium for a pluripotent stem cell (preferably a medium for maintaining undifferentiation) can be used as the medium used in the step of maintaining undifferentiation, and a component that suppresses apoptosis that occurs in dispersing iPS cells may be blended, such as "Y-27632" (CAS RN(R). 331752-47-7; C₁₄H₂₁N₃O•2HCl•H₂O =338.27).

For example, a commonly used culture medium for an epidermal keratinocyte (preferably a differentiation medium) can be used as the medium used in the differentiation induction step, and when initiating differentiation induction, components for initiating differentiation induction of pluripotent stem cells may also be blended.

Furthermore, components for maintaining differentiation of an epidermal keratinocyte may be blended to a medium for differentiating a pluripotent stem cell to an epidermal keratinocyte and maintaining cells (differentiation medium), and although the components for maintaining differentiation are not particularly limited, for example, human epidermal growth factor and Y-27632 are suitable when obtaining an epidermal keratinocyte.

For example, components for initiating differentiation induction (also referred to as "for promoting differentiation induction") include BMP4, fibroblast growth factor (e.g., FGF1), epidermal growth factor (e.g., EGF), ascorbic acid, cholera toxin, nicotinamide, cyclic AMP analogs (e.g., 8-Br-cAMP), TGFβRI kinase inhibitor II (TGFRKi), retinoic acid (e.g., all-trans RA), and the like, and one or two or more selected from these can be used.

In addition, as components for promoting proliferation of an epidermal keratinocyte, for example, available growth factors such as peptide hormones, cytokines, and ligand-receptor complexes; proliferation factors such as generation FGF (FGF1), BMP-4, EGF, etc. can be mentioned, and one or two or more selected from these can be used.

The effective concentration of the component for promoting proliferation or differentiation in this case may be preferably about 0.01 to about 500 ng/mL, more preferably about 0.1 to about 500 ng/mL, or about 1 to 100 ng/mL.

In addition, when culturing single cells (undifferentiation maintenance culture, differentiation induction culture, subculture, cell maintenance culture, etc.), it may be carried out in the presence of small molecules used to increase cloning efficiency and cell survival rate, for example, ROCK inhibitors. Examples of the ROCK inhibitor or ROCK inhibitory component include Y-27632, HA-1077, H-1152, HA-100, and blebbistatin, and one or two or more selected from these can be used. The effective concentration of the component in this case is, for example, at least or about 0.02, 0.05, 0.1, 0.2, 0.5, 1, 2, 3, 4, 5, 10, 15, 20, 25, 30, 35, 40, 45, 50 to about 100 µM, or any concentration derived from this range.

### 1-2. Differentiation induction method in the present embodiment

In the present embodiment, it is preferable to include at least a step of inducing differentiation from a pluripotent stem cell to an epidermal keratinocyte. Furthermore, it is preferable to include at least an undifferentiation maintenance step of culturing or subculturing a pluripotent stem cell in an undifferentiated state, and a step of inducing differentiation from the pluripotent stem cell that has undergone the undifferentiation maintenance step to an epidermal keratinocyte. The differentiation induction method in the present embodiment may be a method for producing an epidermal keratinocyte from a pluripotent stem cell.

In the present embodiment, it is preferable to use a non-detached cell after being treated with a cell detaching agent in the inducing step from the pluripotent stem cell when inducing differentiation from a pluripotent stem cell to an epidermal keratinocyte, and to use the non-detached cell in at least one cell culture or subculture in the induction step. As a result, it is possible to obtain a three-dimensional cultured skin model in which the horny cell layer is multi-layered when an epidermal keratinocyte is applied to a three-dimensional cultured skin model and to obtain an epidermal keratinocyte that is used for producing or can produce the three-dimensional cultured skin model in which the horny cell layer is multi-layered. Furthermore, it is also possible to provide a method for producing a three-dimensional cultured skin model by using the epidermal keratinocyte, and a three-dimensional cultured skin model with better quality.

In addition, in the present embodiment, when culturing animal cells, it can be carried out under aseptic operation in an apparatus and environmental conditions that allow the culturing of animal cells that are commonly used, and for example, in cell culture, animal cell culture equipment (e.g., carbon dioxide gas incubator) that can be controlled so that the pH becomes around neutrality in an environment of 5% CO₂ and a culture temperature of 37°C can be used, and it is preferable that the device can be controlled in an environment of 5% CO₂ and a culture temperature of 37°C, in the differentiation induction step, the three-dimensional cultured skin model production step, and the like.

In addition, in the description of the differentiation induction method in the present embodiment, descriptions of each configuration of the pluripotent stem cell detaching agent, culture substrate, cell culture equipment, etc., and each treatment method, etc., which overlap with the above-mentioned "1-1." etc. are omitted as appropriate, but the description of "1-1." etc. applies also to the present embodiment and can be adopted as appropriate. Furthermore, in the present embodiment, descriptions of "2." and "3." to be mentioned later can also be applied to the present embodiment and can be adopted as appropriate.

### 1-2-1. Undifferentiation maintenance step (pluripotent stem cell maintenance step)

In the present embodiment, a pluripotent stem cell maintenance step may be performed before the differentiation induction step, or the obtained or produced pluripotent stem cell may be used to immediately perform the differentiation induction step with omitting this undifferentiation maintenance step.

In the present embodiment, it is preferable to perform culture to maintain an undifferentiated state for a certain period of time after seeding a pluripotent stem cell, before the differentiation induction step, and since the cell states after initiating differentiation induction (the number and shape of single cells) can be improved, it is possible to more efficiently induce an epidermal keratinocyte that can better produce a three-dimensional cultured skin model with a multi-layered horny cell layer, from a pluripotent stem cell.

In the present embodiment, as a more preferable aspect, the method includes performing culturing for maintaining an undifferentiated state for a predetermined period after seeding a pluripotent stem cell, before the differentiation induction step. It is preferable to perform undifferentiation maintenance culture until the cells reach a logarithmic growth phase, before initiating induction in the differentiation induction step, and in this case, it is more preferable to replace the undifferentiation maintenance medium every day, and the period of logarithmic growth is included in the number of days in the period from "seeding to initiation of induction" in FIG. 1. The predetermined period is preferably 3 to 7 days, more preferably 3 to 6 days, and still more preferably 3 to 5 days. In addition, when the induction initiation date is used as the reference (day 0), the seeding initiation date is preferably day -2 to day -6, more preferably day -3 to day -5 with respect to the induction initiation date (see, Figure 1 section "Seeding - induction initiation").

As the culture medium, a commercially available medium such as StemFit(R) AK02N can be used, and during the period of the undifferentiation maintenance step, it is preferable to replace the culture medium as appropriate, preferably every 1 to 2 days, more preferably every day. The culture medium may contain an added component that suppresses apoptosis of a pluripotent stem cell, such as Y-27632 and the like. In addition, as the culture substrate (coating agent) and cell culture equipment, the above "1-1-2." etc. can be adopted as appropriate, and laminin 511E8 fragment is more preferred, and polystyrene cell culture equipment is more preferred.

In addition, in order to make the cell state (number and shape of single cells, etc.) more suitable and perform the next step of inducing differentiation, culturing may be performed multiple times in the undifferentiation maintenance step, and for example, "Culture after subculture operation" may be carried out singly or in plurals. The culture period is not particularly limited, but the period and number of times used in cell culture for maintaining a pluripotent stem cell are suitable, and for example, after culturing the cells (for example, about 6 to 8 days), cell detachment is performed using a cell detaching agent, and culturing is performed with the detached cells, and the like. As the cell detaching agent used in the pluripotent stem cell maintenance step, commonly used components can be used, and for example, the above "1-1-3." etc. can be adopted as appropriate, and protease is preferable, and more preferable are one or two or more selected from trypsin, trypsin-like protease, trypsin-like protease derived from microorganisms, and TrypLE (trademark) select enzyme, and still more preferable are trypsin, gene recombinant fungus-derived trypsin-like protease, and TrypLE (trademark) select enzyme, and commercially available products include, for example, cell detaching agents used in [Examples] below.

Cells may be treated with a ROCK inhibitor (e.g., Y-27632, etc.) or a myosin II inhibitor before and/or after cell detachment.

### 1-2-2. Differentiation induction step

In the differentiation induction step in the present embodiment, it is preferable to induce differentiation from a pluripotent stem cell to an epidermal keratinocyte. As a more preferred embodiment, it is preferable to culture a pluripotent stem cell to initiate or promote differentiation induction in a differentiation induction initiation medium, and/or to perform culture or subculture to maintain differentiation or promote differentiation of a pluripotent stem cell after the initiation of differentiation induction in a differentiation maintenance medium. Furthermore, in culturing after culture or subculture operation for maintaining differentiation or promoting differentiation, it is preferable to use detached cells obtained by detaching cells that have reached confluence using a cell detaching agent, in the next culture.

As a preferred embodiment of the present embodiment, it is preferable to perform the culture step in the differentiation induction step or the culture after the subculture operation multiple times.

In addition, as a more preferred aspect of the present embodiment, when the culture step in the differentiation induction step or the culture step after subculture operation is performed multiple times, a culture medium (preferably a medium for maintaining differentiation) is added to non-detached cells that have not been detached from the cell culture equipment using a cell detaching agent and are still attached to the cell culture equipment, and then culturing is performed continuously, and more preferably, a culture medium is added to the cell culture equipment with attaching non-detached cells, and then culturing is performed subsequently, in at least one culture step of culture step after subculture operation.

In a further preferred embodiment, it is preferable to include
a culture step in which in a differentiation induction step, pluripotent stem cells are cultured in a differentiation induction initiation medium and a differentiation maintenance medium, and detached cells detached using a cell detaching agent are collected (first culture step) (e.g., P=0 in FIG. 1),
a culture step in which the detached cells are cultured in a differentiation maintenance medium, and non-detached cells that cannot be detached from the cell culture equipment using a cell detaching agent during subculturing operation and are adhered to the cell culture equipment are kept (second culture step) (e.g., P=1 remaining in Figure 1: subculture day 25),
a culture step in which a culture medium (preferably a differentiation maintenance medium) is added to the non-detached cell, culturing is performed subsequently, and the detached cells that were detached using a cell detaching agent during the subculture operation are collected (third culture step) (e.g., P=1 remaining in Figure 1: subculture day 29), and
a culture step in which the detached cells are cultured in a differentiation maintenance medium, and the detached cells detached using a cell detaching agent are collected (fourth step) (e.g., P=2-B). Note that the detached cells collected in the fourth culture step can be used as an epidermal keratinocyte derived from a pluripotent stem cell. Note that it is preferable to use the cell detaching agent after culturing the cells and reaching confluence. In addition, in the present specification, the terms first, second, third, ..., n-th are given in order for convenience of description, and these do not limit the culture conditions in the present embodiment (for example, the number of times of culture, addition or insertion of other steps, etc.).

In the present embodiment, in the step of inducing from a pluripotent stem cell, it is preferable to use non-detached cells that have been treated with a cell detaching agent, and to use the non-detached cells for cell culture or subculture at least once in the inducing step.

The cell detaching agent used to obtain the non-detached cell can be appropriately selected from the above "1-1-3." and the like, and a cell detaching agent containing protease is preferable, and the protease includes preferably one or two selected from trypsin, trypsin-like protease, trypsin-like protease derived from microorganisms, trypsin-like protease derived from gene recombinant fungi, and TrypLE (trademark) select enzyme, and more preferably trypsin, trypsin-like protease derived from gene recombinant fungi, or TrypLE (trademark) select enzyme. Further, the cell detaching agent for obtaining the non-detached cell may be a cell detaching agent containing TrypLE (trademark) select enzyme, or a cell detaching agent containing the TrypLE (trademark) select enzyme and 0.5 to 1.5 mM EDTA, and instead of the TrypLE (trademark) select enzyme, any one or a combination of trypsin, trypsin-like protease, microorganism-derived trypsin-like protease, or gene recombinant fungus-derived trypsin-like protease may be added. In the present embodiment, proteases, cell detaching agents, etc. can be used to detach cells, and can also be used in obtaining a non-detached cell.

The non-detached cells are preferably cells that have been treated with a culture substrate on the cell culture equipment before being treated with the cell detaching agent, then seeded and cultured, and then treated with the cell detaching agent.

Further, it is preferable that the non-detached cells are cultured in a medium for maintaining differentiation (more preferably a medium for maintaining differentiation containing a ROCK inhibitor and an epithelial cell factor), and in this case, the cells are cultured until they reach confluence. Furthermore, in the present embodiment, after the culture until reaching confluence, it is more preferable to treat with a cell detaching agent and use the detached cells detached with the cell detaching agent, in the next culture.

The culture substrate contained in the coating agent used to obtain the non-detached cell can be appropriately selected from the above "1-1-2." and the like, and among these, laminin or a fragment thereof is preferable, a laminin fragment is more preferable, and a laminin 511E8 fragment is even more preferable. Further, the cell culture equipment can be appropriately selected from the above-mentioned "1-1-2."and the like, and cell culture equipment made of polystyrene is more preferable.

Furthermore, it is preferable that the non-detached cell is based on detached cells collected after culturing one or more times after the initiation of induction and performing detachment treatment with a cell detaching agent during the culture. Furthermore, it is preferable to perform the next culture using the non-detached cell.

In the differentiation induction step, in order to stably induce differentiation of a pluripotent stem cell, it is preferable to perform culturing multiple times.

In a typical cell culture aspect, once the cell culture equipment is full, the colony is divided into aggregated cells or single cells using any method suitable for detachment, and these are placed in a new cell culture equipment for subculturing, and culture is performed. This culturing is a technique for keeping cells viable and allowing them to proliferate for long periods of time under culture conditions. Cells are usually then cultured when they reach about 70-100% confluence. The confluence is more preferably 90% or more, still more preferably 100% or more.

The number of times of culturing after the subculture operation when culturing in the differentiation induction step is not particularly limited, but is preferably multiple times, more preferably 2 to 5 times, even more preferably 2 to 4 times, and still more preferably three times.

Further, in the differentiation induction step, it is usually desirable to carry out the culture three times after the subculture operation. In the present embodiment, it is preferable to continue culturing the non-detached cell obtained during the second subculture operation, and perform the third subculture operation when the cells reach confluence. It is preferable to obtain the confluent epidermal keratinocyte by culturing after the third subculture operation, and to provide this epidermal keratinocyte to a three-dimensional cultured skin model. The culture medium and culture period in this case may be the same as those in the differentiation induction step described above.

In the differentiation induction step, when using the non-detached cell, it is preferable to use the non-detached cell in one or two cultures prior to the culture for collecting an epidermal keratinocyte for the production of a three-dimensional cultured skin model, and more preferably, the non-detached cell is used in the previous culture.

Furthermore, after culturing is performed using the non-detached cell, the cells used in the next culture are preferably detached cells that have been detached using a cell detaching agent.

In the differentiation induction step, the cell detaching agent used to obtain detached cells is preferably a component of a normal cell detaching agent, and the components are preferably one or two selected from trypsin, trypsin-like protease, trypsin-like protease derived from gene recombinant microorganisms, and TrypLE (trademark) select enzyme, and trypsin-based enzymes are more preferable, and further, trypsin, trypsin-like protease derived from gene recombinant fungi, or TrypLE (trademark) select enzyme is further preferred.

In addition, in the differentiation induction step, a normal coating agent can be used as a coating agent to coat the cell culture equipment when obtaining detached cells, but the culture substrate contained in the coating agent is preferably laminin or its fragment, more preferably a laminin fragment, still more preferably a laminin 511E8 fragment. Further, the material of the cell culture equipment in this case is preferably plastic, more preferably a styrene resin (more preferably polystyrene).

As for the culture environment conditions in the differentiation induction step, it is preferable to carry out the culture under an environment of, for example, 5% CO₂ and a culture temperature of 37°C. In addition, the culture medium used in the differentiation induction step is not particularly limited, but it is preferable that components for differentiation induction are added to the culture medium at the initiation of differentiation induction, and retinoic acid and BMP-4 are more preferable, and it is preferable that components for maintaining differentiation have been added to the culture medium during the period of maintaining differentiation.

Preferred aspects of the present embodiment include
a step in which in the differentiation induction step, after the initiation of induction, culture (first culture) is performed and when reaching confluence, the cells detached with a cell detaching agent are collected;
a step in which when culture (second culture) is performed based on the collected detached cells and reaches confluence, non-detached cells that have not been detached with a cell detaching agent are left in the cell culture equipment;
a step in which culture is performed until reaching confluence based on the remaining non-detached cells, and cells detached with a cell detaching agent are collected; and
a step in which when culture (third culture) is performed based on the collected detached cells and reaches confluence, cells detached with a cell detaching agent are collected.

Furthermore, it is more preferable to use the cells finally collected (for example, during third culture) as the epidermal keratinocyte in the present embodiment, for producing a three-dimensional cultured skin model.

In addition, in the description of the method for inducing differentiation from a pluripotent stem cell to an epidermal keratinocyte in the present embodiment, each configuration and each treating method of the pluripotent stem cell detaching agent, culture substrate, cell culture equipment, and the like, which overlap with "2." etc. described later, will be omitted as appropriate, but the descriptions also apply to the present embodiment and can be adopted as appropriate.

### 2. Method for producing epidermal keratinocyte from pluripotent stem cell in the present embodiment

In the description of the method for producing an epidermal keratinocyte in the present embodiment, descriptions of each configuration and each treating method of the pluripotent stem cell detaching agent, culture substrate, cell culture equipment, and the like, which overlap with "1." etc. described above, will be omitted as appropriate, but the descriptions of "1." etc. also apply to the present embodiment and can be adopted as appropriate. Furthermore, in the present embodiment, the descriptions of "3." etc. to be described later can also be applied to the present embodiment and can be adopted as appropriate.

Another embodiment of the present invention may also provide a method for producing an epidermal keratinocyte from a pluripotent stem cell, characterized by inducing differentiation from a pluripotent stem cell to an epidermal keratinocyte using a non-detached cell after being treated with a cell detaching agent, in the induction step from a pluripotent stem cell, for obtaining a three-dimensional cultured skin model in which the horny cell layer is stratified when the epidermal keratinocyte is applied to a three-dimensional cultured skin model.

Furthermore, another embodiment of the present invention may also provide a method for producing an epidermal keratinocyte, including a step of inducing differentiation from a pluripotent stem cell to an epidermal keratinocyte using a non-detached cell after being treated with a cell detaching agent. The epidermal keratinocyte is a cell that can be better derived from a pluripotent stem cell and is in good cell condition, and has a merit that a three-dimensional cultured skin model with a multi-layered horny cell layer can be produced more successfully.

In the present embodiment, epidermal keratinocytes in good condition or an epidermal keratinocyte group containing these can be produced from a pluripotent stem cell in a better and simpler manner. Furthermore, in the present embodiment, a three-dimensional cultured skin model having a multi-layered horny cell layer can be produced more simply, more successfully and stably.

Furthermore, still another embodiment of the present invention may also provide an epidermal keratinocyte produced using the method for inducing differentiation from a pluripotent stem cell to an epidermal keratinocyte. Furthermore, as another embodiment of the present invention, an epidermal keratinocyte sheet may be produced using an epidermal keratinocyte derived from a pluripotent stem cell in the present embodiment, or this epidermal keratinocyte sheet may be provided. The epidermal keratinocyte in the present embodiment and the epidermal keratinocyte sheet can be used for skin-related regenerative medicine and therapeutic purposes for mammals such as humans, and can be used for regenerative medicine, skin transplantation, skin treatment, custom-made products related to skin, etc., but are not limited to these.

### 3. Method for producing three-dimensional cultured skin model in the present embodiment

In the description of the method for producing a three-dimensional cultured skin model in the present embodiment, descriptions of each configuration and each treatment method of the pluripotent stem cell detaching agent, culture substrate, cell culture equipment, etc., which overlap with the above-mentioned "1.", "2.", etc., will be omitted as appropriate, but the descriptions of "1.", "2.", etc. are also applicable to the present embodiment and can be adopted as appropriate. Further, in the present embodiment, the description to be described later can also be applied to the present embodiment and can be adopted as appropriate.

Another embodiment of the present invention may provide a method for producing a three-dimensional cultured skin model using the epidermal keratinocyte; and a three-dimensional cultured skin model obtained by the production method or using the epidermal keratinocyte.

In this case, the method for producing a three-dimensional cultured skin model in the present embodiment can adopt known production methods or culture methods of a three-dimensional cultured skin model, except for using an epidermal keratinocyte in the present embodiment. This embodiment has a merit that a three-dimensional cultured skin model can be efficiently obtained by using an epidermal keratinocyte of the present embodiment even if a known producing method or culture method is employed.

When observing the three-dimensional cultured skin model obtained using an epidermal keratinocyte derived from a pluripotent stem cell using conventional methods, the horny cell layer was not sufficiently multi-layered, and the three-dimensional cultured skin model with good quality could not be formed, thus having a problem in quality. The three-dimensional cultured skin model produced using an epidermal keratinocyte derived from a pluripotent stem cell, which has such quality problem, has poor quality as the three-dimensional cultured skin model (for example, the horny cell layer is discontinuously unformed, etc.), and therefore, when used in skin physiological function tests or in vitro tests, troubles and reproducibility of results frequently occur, making it undesirable.

If a three-dimensional cultured skin model in which the horny cell layer is multi-layered can be created using an epidermal keratinocyte derived from a pluripotent stem cell, it would be possible to perform experiments that could not be conducted with usual epidermal keratinocytes, such as gene editing experiments or reproducing individual skin texture. In the present embodiment, the three-dimensional cultured skin model produced using an epidermal keratinocyte induced to differentiate from a pluripotent stem cell can be said to be a high quality three-dimensional cultured skin model since the horny cell layer is continuously multi-layered over a wide area, and experiments that could not be conducted with usual epidermal keratinocytes, as described above, become possible, and further, skin physiological function tests or in vitro tests that are closer to animal skin can be conducted. Furthermore, a skin physiological function test or an in vitro test using the present embodiment can provide more reliable results, and is also preferable from the viewpoint of animal welfare. Furthermore, in the present embodiment, a three-dimensional cultured skin model can be produced without using spheroids, which will also lead to reductions in labor and cost associated with producing spheroids.

Furthermore, the three-dimensional cultured skin model of the present embodiment has a multi-layered horny cell layer constituting two or more layers, so it has a skin condition closer to that of humans, and further, the multi-layered horny cell layers are formed in a wide range on the plane (X-axis direction). The three-dimensional cultured skin model of the present embodiment is constructed with two layers, wherein the upper layer is a multi-layered horny cell layer based on an epidermal keratinocyte derived from a pluripotent stem cell of the present embodiment, and the lower layer is constituted to be made of a collagen layer embedded with fibroblasts, fabricated resembling the structure of human skin. The origin of the fibroblasts is not particularly limited, and may be derived from mammals (preferably humans), and somatic fibroblasts (for example, human skin fibroblasts) or fibroblasts derived from pluripotent stem cells may be used, more preferably human dermal fibroblasts are used.

The three-dimensional cultured skin model of the present embodiment can be produced by appropriately adopting a known method for producing a three-dimensional cultured skin model, except for using an epidermal keratinocyte derived from a pluripotent stem cell of the present embodiment as the raw material for the upper layer.

An example of a method for producing a three-dimensional cultured skin model in the present embodiment is shown below, but the present embodiment is not limited thereto. When producing a three-dimensional cultured skin model using an epidermal keratinocyte derived from a pluripotent stem cell of the present embodiment, it can be carried out under general culture conditions using cell culture equipment such as a general cell culture plate or the like. A culture container such as a culture insert for producing a three-dimensional cultured skin model can be used, and examples of the culture container include cell culture inserts for skin tissue culture, and the like. The material of the culture container is not particularly limited, and examples thereof include polycarbonate and the like. One or two or more selected from these can be used.

Supports used for other than culture inserts are not particularly limited, and include, for example, collagen gels, collagen sponges, acellular dermis from which cells have been removed, and those incorporating dermal fibroblasts as feeder cells, and the like, and one or two or more selected from these can be selected, and it is preferable to use collagen gel.

Examples of known methods for producing three-dimensional cultured skin models include, but are not limited to, collagen gel culture methods. Examples of the collagen gel culture method include a collagen gel embedding method (three-dimensional culture), a method of culture on a collage gel, a collagen coating method, etc., and one or two or more can be selected from these.

A preferred aspect of the production method of the present embodiment preferably includes at least a step of culturing an epidermal keratinocyte, in which an epidermal keratinocyte derived from a pluripotent stem cell of the present embodiment is seeded and cultured on a collagen gel containing fibroblasts. Furthermore, a more preferred embodiment preferably includes at least a collagen gel producing step for producing a collagen gel containing fibroblast, and a step of culturing an epidermal keratinocyte in which an epidermal keratinocyte derived from a pluripotent stem cell of the present embodiment is seeded and cultured on a collagen gel containing fibroblasts. Furthermore, after the culturing step, the medium covering the layer of an epidermal keratinocyte is removed and the epidermal keratinocyte is exposed to air, and after a certain period of time (preferably 12 to 14 days) has elapsed, a three-dimensional cultured skin model can be obtained.

The culture conditions can be selected as appropriate from conditions that allow the animal cells of origin to be cultured, and in the case of culture temperature, for example, in the case of human origin, the preferred temperature is about 37°C, i.e. 36 to 38°C, and in an atmosphere in culturing, the carbon dioxide concentration in the atmosphere may be, for example, 2 to 5% or 5% CO₂ concentration, and the oxygen concentration in the atmosphere may be, for example, 3 to 20% O₂ concentration, although it is not particularly limited, and additionally, regarding conditions other than these, the atmosphere may be adjusted appropriately with air or nitrogen gas. Moreover, as the culture medium to be used, commercially available products suitable for each process and each period can be appropriately adopted.

The number of cells to be seeded is preferably 10³ to 10⁶ cells, more preferably 1 to 5× 10⁵ cells per well.

The three-dimensional cultured skin model of the present embodiment is suitable in that it is embedded after tissue fixation and sucrose replacement, cut into 10 µm thick sections, and in HE staining and microscopic observation, when observed from the side so that two layers, the fibroblast layer and the epidermal keratinocyte layer, in the section are visible, the continuous multi-layered part of the horny cell layer that is stained red with eosin is formed in 80% or more in the horizontal axis direction of the skin section. The range of the continuous multi-layered horny cell layer is more preferably 90% or more, still more preferably 95% or more, and even more preferably 100%.

The pass/fail judgment of the three-dimensional cultured skin model of the present embodiment can be performed according to <Pass/Fail judgment of three-dimensional cultured skin model> described in [Examples] below.

In the method for producing a three-dimensional cultured skin model in the present embodiment, an epidermal keratinocyte that can be better derived from a pluripotent stem cell can be used, and therefore, there is an advantage that a three-dimensional cultured skin model having a multi-layered horny cell layer can be produced successfully even if a general method for producing a three-dimensional cultured skin model is used.

The three-dimensional cultured skin model of the present embodiment can be used as an in vitro test or skin model that is more similar to mammals such as humans, for example, for searching for target active ingredients, screening test compounds, examining test compositions, and the like.

The mammal in the present embodiment is not particularly limited, and includes humans, pigs, cows, mice, rats, guinea pigs, hamsters, rabbits, etc., and one or two or more selected from these can be used. Among these, humans and pigs are preferred, and humans are more preferred.

The three-dimensional cultured skin model of the present embodiment can also be used as an experimental model (skin model) as an alternative method to animal experiments.

In addition, the three-dimensional cultured skin model of the present embodiment can be produced by creating a pluripotent stem cell from somatic cells collected from subjects such as healthy people and patients, and using an epidermal keratinocyte derived from the pluripotent stem cell, and therefore, it can also be used for techniques applied to the target person (custom-made cosmetics, skin care, etc.). The three-dimensional cultured skin model produced by the method of the present embodiment from a pluripotent stem cell produced from the subject's cells can also be used as one that is closer to the subject's own skin (skin condition, metabolic mechanism, etc.). Therefore, it can be used for determining the appropriateness of active ingredients, predicting usage amounts, side effects, etc., and preventing, improving, or treating skin conditions of subjects. Furthermore, by creating a three-dimensional cultured skin model using the method of the present embodiment from a pluripotent stem cell in which specific genes have been gene-edited, it is also possible to analyze the functions of specific genes in the skin.

By administering (e.g. applying) drugs, external skin preparations, cosmetics, active ingredients, effective compositions, etc. as test substances to the three-dimensional cultured skin model of the present embodiment, and comparing them with controls (no additives, positive, negative, etc.), evaluation, investigation, screening, etc. of the drug, etc. can be carried out.

The evaluation method, screening method or the like of the present embodiment may include a step of administering a test substance to the three-dimensional cultured skin model and culturing it, and a step of determining the suitability of the test substance from the state of the three-dimensional cultured skin model cultured after administration. As the culture conditions for the three-dimensional cultured skin model in this case, known culture conditions for a three-dimensional cultured skin model can be adopted. For example, the test can be conducted in a cell culture incubator under conditions of 3-6% CO₂, 3-25% O₂ and 36-38°C.

The method of administration is not particularly limited, and suitable methods include application, transdermal administration, injection administration, etc., and more preferably, application to the skin or transdermal administration.

A more preferred aspect of the administration method is application to three-dimensionally cultured skin, which includes, but is not limited to, application by painting, application by spraying, and application by permeability such as pasting on the skin.

Note that the present embodiment may be used for therapeutic purposes or non-therapeutic purposes. "Non-therapeutic purpose" is a concept that does not include medical actions (for example, actions performed by a doctor), that is, actions that treat the human body through therapy. Examples of non-therapeutic purposes include beauty, makeup, and the like.

Furthermore, in the present embodiment, "prevention" refers to preventing or delaying the onset of a symptom or disease in a subject, reducing the risk of onset of a symptom or disease in a subject, or the like. In the present technique, "improvement" refers to recovery or maintenance of a disease, symptom, or condition in a subject; prevention or delay of deterioration; reversal, prevention, or delay of progression.

The present technique can employ the following configurations or technical features [1] to [12].
[1] A method for inducing differentiation from a pluripotent stem cell to an epidermal keratinocyte, using a non-detached cell after being treated with a cell detaching agent in the inducing step from the pluripotent stem cell,
   wherein when applying the epidermal keratinocyte to a three-dimensional cultured skin model, a three-dimensional cultured skin model in which the horny cell layer is multi-layered is obtained.
[2] The method for inducing differentiation from a pluripotent stem cell to an epidermal keratinocyte according to [1] above, wherein the induction is initiated 3 to 6 days after seeding the pluripotent stem cell.
[3] The method for inducing differentiation from a pluripotent stem cell to an epidermal keratinocyte according to [1] or [2] above, wherein the cell detaching agent contains one or two or more selected from trypsin and trypsin-like proteases.
[4] The method for inducing differentiation from a pluripotent stem cell to an epidermal keratinocyte according to [1] or [2] above, wherein the cell detaching agent contains trypsin and/or trypsin-like protease derived from a microorganism.
[5] The method for inducing differentiation from a pluripotent stem cell to an epidermal keratinocyte according to any one of [1] to [4] above, wherein the non-detached cell is a cell that has been obtained by treating the surface of the cell culture equipment with a culture substrate, then seeding and culturing the cell, before treating with the cell detaching agent, and then treating with the cell detaching agent.
[6] The method for inducing differentiation from a pluripotent stem cell to an epidermal keratinocyte according to any one of [1] to [5] above, wherein
   the non-detached cell is a cell that has been obtained by treating the surface of the cell culture equipment with a culture substrate, then seeding and culturing the cell, before treating with the cell detaching agent, and then treating with the cell detaching agent, and
   the culture substrate is laminin or a fragment thereof.
[7] The method for inducing differentiation from a pluripotent stem cell to an epidermal keratinocyte according to any one of [1] to [6] above, wherein the non-detached cell is based on detached cells collected after culturing after initiation of the induction and performing detachment treatment with a cell detaching agent during the culturing.
[8] The method for inducing differentiation from a pluripotent stem cell to an epidermal keratinocyte according to any one of [1] to [7] above, wherein the non-detached cell is used for the next culturing.
[9] The method for inducing differentiation from a pluripotent stem cell to an epidermal keratinocyte according to any one of [1] to [8] above, wherein the pluripotent stem cell is an artificial pluripotent stem cell.
[10] The method for inducing differentiation from a pluripotent stem cell to an epidermal keratinocyte according to any one of [1] to [9] above, wherein
   the pluripotent stem cell is an artificial pluripotent stem cell, and
   the artificial pluripotent stem cell is an artificial pluripotent stem cell derived from epidermal keratinocytes.
[11] A method for producing an epidermal keratinocyte, including a step of inducing differentiation from a pluripotent stem cell to an epidermal keratinocyte, using a non-detached cell after being treated with a cell detaching agent.
[12] A method for producing a three-dimensional cultured skin model, using an epidermal keratinocyte produced using the method for inducing differentiation from a pluripotent stem cell to an epidermal keratinocyte according to any one of [1] to [11] above.

### EXAMPLES

The present technique will be described in more detail below based on examples and the like. Note that the examples and the like described below are examples of typical embodiments of the present technique, and the scope of the present technique should not be interpreted narrowly thereby.

### <Test Example 1: Examination of condition for inducing differentiation from pluripotent stem cell to epidermal keratinocyte>

Regarding the examination of conditions for inducing differentiation from a pluripotent stem cell to an epidermal keratinocyte in Test Example 1, <Test Example 1-1: Examination of the type of iPS cells used>, <Test Example 1-2: Examination of the type of coating agent (culture substrate) for plate (cell culture equipment)>, <Test Example 1-3: Examination of optimization of the period from seeding to initiation of induction>, <Test Example 1-4: Examination of selection of detached cells/non-detached cell> and <Test example 1-5: Examination of the type of cell detaching agent during subculture operation> were carried out.

### <Production Example 1. Step of maintaining undifferentiated iPS cell culture>

Human keratinocyte-derived iPS cells (obtained from Nihon Pharmaceutical University) were cultured in StemFit (R) AK02N (Ajinomoto Healthy Supply Co., Inc.) at 37°C and 5% CO₂. For culture, a polystyrene tissue culture plate coated with iMatrix-511 silk (produced and developed by Nippi, Inc., sold by Matrixome Inc., a culture substrate made by purifying human laminin 511E8 fragment) was used. The medium was replaced with StemFit (R) AK02N every day. iPS cells were detached every 7 days for subculturing with TrypLE (trademark) Select Enzyme (cell detaching agent) (Gibco; a product containing a recombinant cell dissociation enzyme (also referred to as "cell detachment enzyme") to replace trypsin):PBS = 1:1 solution and cultured for 1 day in StemFit (R) AK02N supplemented with 1 µM Y-27632 (Wako).

Note that the human keratinocyte-derived iPS cells were produced using human keratinocytes, which are differentiated cells, with reference to <Okita K. et. al., Nature Methods, 2011, 8:409-12.>.

StemFit (R) AK02N (medium) is an animal-derived component-free (Xeno-free) and feeder-less (or feeder-free) medium, and it is a culture medium for maintaining undifferentiation that attains undifferentiation maintenance culture for proliferating while maintaining undifferentiated nature of human pluripotent stem cells (ES cells, iPS cells) and is also suitable for maintaining the ability to differentiate into keratinocytes. Note that feeder cells refer to other cell types that play an auxiliary role and are used to adjust culture conditions for target cells whose proliferation and differentiation are to occur. Usually, feeder cells are treated in advance with gamma ray irradiation or antibiotics to prevent them from proliferating.

"Y-27632" (CAS RN(R). 331752-47-7; C₁₄H₂₁N₃O•2HCl•H₂O =338.27)" supplemented to StemFit (R) AK02N is a selective and strong ROCK inhibitor, and can be used to suppress cell death during cell dispersion of pluripotent stem cells and to improve cell survival rate after cryopreservation.

iMatrix-511 silk is a recombinant human laminin 511-E8 protein expressed from gene recombinant silkworm cocoons, and is a culture substrate that exhibits binding activity with integrin α6B1 with a dissociation constant of 10 nM or less.

TrypLE (trademark) Select Enzyme (cell detaching agent) is a non-animal alternative to porcine trypsin, and contains recombinant enzyme (specifically trypsin-like proteases) derived from microbial fermentation (specifically fungi), and the enzyme has a removal rate and recovery survival rate comparable to those of porcine trypsin, and the product is a 1X stock solution formulated in DPBS (Dulbecco's Phosphate Buffered Saline) containing 1 mM EDTA.

### <Production Example 2. Differentiation of human keratinocyte-derived iPS cell to epidermal keratinocyte>

Human keratinocyte-derived iPS cells were grown in the above-described undifferentiation maintenance culture and seeded on polystyrene tissue culture plates coated with iMatrix-511 silk, and the medium was then replaced every day with StemFit (R) AK02N. Once in the logarithmic growth phase, the culture was replaced with CnT-07 (CELLnTEC) medium supplemented with 1 µM retinoic acid (Sigma-Aldrich) and 25 ng/mL BMP-4 (Wako), for inducing differentiation to an epidermal keratinocyte, and cultured for 4 days at 37°C under 5% O₂ and 5% CO₂. Thereafter, the medium was replaced with CnT-07 (CELLnTEC) medium (hereinafter referred to as differentiation maintenance medium) supplemented with 20 ng/mL EGF (MACS) and 10 µM Y-27632 (initiation of induction: day 0 of culture: P=0). Thereafter, the medium was replaced every day.

The number of culture days from seeding to the initiation of induction was 5 days.

When approximately 2 weeks passed after switching to the differentiation maintenance medium and the entire surface of the coated polystyrene tissue culture plate was covered with cells, that is, when the cell confluence reached 100%, subculture was performed (P=1). Subculture involves treating cells with TrypLE (trademark) Select Enzyme (cell detaching agent), separating the detached cells by centrifuging them, suspending the cells in differentiation maintenance medium, and seeding on a new tissue culture plate.

When the cell confluence reached 100% again, the cells were treated with TrypLE (trademark) Select Enzyme (cell detaching agent), and the cells detached by pipetting (detached cells) were seeded onto a new tissue culture plate (detached cell line P=2-A). At that time, cells that did not detach from the coated polystyrene tissue culture plate even after pipetting (non-detached cell) were added with a medium (CnT-07 medium supplemented with 20 ng/mL EGF and 10 µM Y-27632) and cultured subsequently (non-detached cell line P=1 remaining).

In addition, the pipetting operation at this time is to use a 1 mL pipette and repeat suction and injection approximately 1 to 5 times to generate a water stream in the culture medium, detach the cells with the water stream, and aspirate and collect the detached cells.

When cell confluence reached 100% for both the detached cell line (P=2-A) and the non-detached cell line (P=1 remaining), the cells detached with the same TrypLE (trademark) Select Enzyme (cell detaching agent) as above were subcultured, respectively (P=3, P=2-B). After detachment, cells of the detached cell line (P=3) and cells of the non-detached cell line (P=2-B) were cultured and proliferated, respectively. The cells of thus produced detached cell line (P=3) and non-detached cell line (P=2-B) were used to create a skin model. Note that differentiation induction includes all processes initiating from day 0.

Note that CnT-07 medium is an epithelial growth medium.

"CnT-07 (CELLnTEC) medium supplemented with 1 µM retinoic acid and 25 ng/mL bone morphogenetic factor 4 (BMP-4)" is a medium used as a medium for inducing differentiation to an epidermal keratinocyte.

"CnT-07 (CELLnTEC) medium supplemented with 20 ng/mL epidermal cell growth factor (EGF (MACS(R) GMP recombinant human EGF)) and 10 µM Y-27632" is a differentiation maintenance medium used as a medium for differentiation of a pluripotent stem cells to an epidermal keratinocyte and for cell maintenance, and is completely synthetic and animal-derived component-free (without any animal-derived or human-derived component) differentiation maintenance medium.

### <Production Example 3. Preparation of skin model>

### <Production Example 3-1. Preparation of fibroblast-embedded collagen gel>

Under ice-cooling, 0.3% Cellmatrix Type I-A (Nitta Gelatin Inc.) (solution A), 5x concentrated DMEM medium (solution B), and 50 mmol/L NaOH solution (solution C) containing 260 mmol/l NaHCO₃ and 200 mmol/L HEPES (4-(2-(hydroxyethyl)-1-piperazineethanesulfonic acid) (Dojindo Laboratories) were mixed at a ratio of 7:2:1, and then, 1 mL each was dispensed into a 6-well multiplate, and gelled at room temperature (20°C). Next, the collected normal human skin fibroblasts (KF-4009: neonatal foreskin skin fibroblasts, obtained from Kurabo Industries) were suspended in B to a concentration of 5×10⁵ cells/mL, and then A:B:C were mixed at a ratio of 7:2:1, and 2 mL each was dispensed onto the previously gelled collagen gel, and gelled at 37°C. DMEM medium supplemented with 10% FBS was added on top of the gel. After 24 hours, the gel was detached from the plate, and the medium was replaced every day thereafter. After 7 days had passed, the next step was carried out.

Note that "Cellmatrix Type I-A" is acid-soluble Type-I collagen derived from pig tendon.

The cell number was calculated using an automatic cell counter.

The skin model was produced at 37°C under 5% O₂ and 5% CO₂ unless otherwise specified.

### <Production Example 3-2. Seeding of keratinocyte>

The keratinocytes used for seeding were iPS cell-derived epidermal keratinocytes of detached cell line (P=3) and iPS cell-derived epidermal keratinocytes of non-detached cell line (P=2-B) obtained in <Production Example 2> above, respectively, and two types of three-dimensional cultured skin models were cultured at the same time.

Specifically, a cell culture insert for skin tissue culture (Greiner, 0.4 µm pore diameter: polycarbonate cell culture equipment) was placed in a 6-well multiplate, and the collagen gel was placed on the insert. A cloning ring with an inner diameter of 10 mm was placed on top of the collagen gel, and 2×10⁵ cells of the collected iPS cell-derived epidermal keratinocytes were seeded inside the ring and cultured in CnT-07 medium containing 1 µM Y-27632. CnT-07 medium was added to the outside of the insert. Twenty four (24) hours after seeding, the medium inside the glass ring was replaced with CnT-07 medium supplemented with 1.73 mM CaCl₂, and the medium outside the insert was replaced with a medium composed of CnT-07 medium supplemented with 1.73 mM CaCl₂ : DMEM medium supplemented with 10% FBS = 1: 1. After 16 to 20 hours, the medium inside the cloning ring was removed, and the epidermal keratinocytes were exposed to air, and the medium outside the insert was replaced. Thereafter, the medium outside the insert was replaced every day.

### <Production Example 3-3. Evaluation of skin model>

12-14 days after air exposure of an epidermal keratinocyte, the cloning ring was removed and the skin model was soaked in 4% PFA-PBS. After 10-12 hours, sucrose replacement was performed by immersing the skin model in a 20% sucrose solution and 9 hours later in a 30% sucrose solution. The skin model was embedded, cut into 10 µm thick sections, and subjected to HE staining (hematoxylin and eosin staining), and the dermis, epidermis, and horny cell layer were observed using an optical microscope. Those in which the horny cell layer, which is stained red with eosin, was formed over more than 80% of the upper part of the skin model when observed were considered to be passed, and those with lower values were considered to be failed.

### <Pass/failure judgment of three-dimensional cultured skin model>

Regarding the pass/fail judgment of the three-dimensional cultured skin model, after sucrose substitution, the three-dimensional cultured skin model was cut vertically using a frozen section preparation device to obtain 10 µm thick sections, and HE staining and microscopic observation were carried out, and it was observed from the side direction so that two layers, the fibroblast layer and the horny cell layer in the section were visible. In the captured image after imaging, in the HE-stained horny cell layer, the lengths of both ends of the three-dimensional cultured skin model on the imaging screen were measured in the X-axis direction (vertical horizontal direction), while the length of the continuously multi-layered horny cell layer portion in the imaging screen was measured.

The range of the multi-layered horny cell layer (X-axis direction) can be determined by the distance of the continuously multi-layered horny cell layer (X-axis (horizontal) direction) in observation of the three-dimensional cultured skin model from the side direction/(distance of the both end (X-axis (vertical) direction) of the three-dimensional cultured skin model in observation of the three-dimensional cultured skin model from the side direction) × 100 (%), and the range of multi-layered horny cell layer (X-axis direction) of 80% or more is passed. The three-dimensional cultured skin model was divided into three equal parts from above in the Y-axis direction and cut in the Z-axis (vertical) direction to obtain three pieces, and when all three of the results of lateral observation of the three HE-stained pieces were passed (∘), it was judged that the three-dimensional cultured skin model passed.

It is an example figure showing the evaluation criterion for pass/fail of a three-dimensional cultured skin model in the present embodiment, and is a cross section in the vertical direction when the three-dimensional cultured skin model is cut in the vertical direction and observed with an optical microscope. The left figure is an example of a failure (×) in which no horny cell layer was formed and the range of multi-layered horny cell layer (X-axis direction) was 0%. The central figure is an example of a failure (×) in which multi-layering of the horny cell layer is observed only within a predetermined range (X-axis direction), and the range of multi-layered horny cell layer (X-axis direction) is 30%. The right figure is an example of a pass (∘) in which multi-layering of the horny cell layer is observed over a predetermined range (X-axis direction), and the range of multi-layered horny cell layer (X-axis direction) is 100%.

### <Test Example 1-1: Examination of iPS cell used (Test Example 1-4: Examination of selection of detached cell/non-detached cell)>

Using the non-detached cell line iPS cell-derived epidermal keratinocytes produced from keratinocyte-derived iPS cells obtained in <Production Example 1> to <Production Example 2>, a three-dimensional cultured skin model was produced according to <Production Example 3. Preparation of skin model>.

As shown in Figure 2, in the three-dimensional cultured skin model based on iPS cells derived from keratinocytes, the horny cell layer is multi-layered throughout the skin, and the thickness of the multi-layering is also large, and thus, a three-dimensional skin culture model having a horny cell layer and a dermis layer could be produced. That is, by using non-detached cell line iPS cell-derived epidermal keratinocytes produced from keratinocyte-derived iPS cells, the objective of obtaining a three-dimensional cultured skin model in which the horny cell layer is multi-layered, when the epidermal keratinocyte is applied to a three-dimensional cultured skin model, could be obtained.

As a result, it could be confirmed that a three-dimensional cultured skin model in which the horny cell layer is multi-layered can be obtained with good reproducibility, when an epidermal keratinocyte is applied to a three-dimensional cultured skin model, by using a pluripotent stem cell such as iPS cells and using a non-detached cell after being treatment with a cell detaching agent in the induction step from a pluripotent stem cell. Furthermore, it could be confirmed that a technique for inducing such a epidermal keratinocyte from a pluripotent stem cell could be provided since a three-dimensional cultured skin model with a multi-layered horny cell layer could be produced by using a non-detached cell during the culture period.

Furthermore, it was supposed that it is preferable to use cells that were not detached even when treated with a cell detaching agent at least once, as the non-detached cell line in conducting subculture using a cell detaching agent several times in culturing in the induction step.

Furthermore, among pluripotent stem cells, iPS cells are preferable, and it is considered that iPS cells derived from keratinocytes are preferable.

<Test Example 1-2: Examination of the type of coating agent (culture substrate) for plate (cell culture equipment)>

Furthermore, regarding the coating agent (culture substrate) used, the above <Production Example 1> to <Production Example 2> were performed, except that Vitronectin or Vitronectin+Col I was used instead of iMatrix. As a result, the state of cells during the differentiation induction step was best for the iMatrix reagent containing laminin 511E8 fragment (extracellular matrix) among these three types of coating agents (culture substrates) used for cell culture equipment (made of polystyrene). As shown in Figure 3, by using laminin or its fragments in the differentiation step from a pluripotent stem cell to an epidermal keratinocyte, the shape and proliferation rate of the cells were good when observed with an optical microscope, and furthermore, the cells survived successfully also in the subsequent subculture.

Therefore, it was confirmed that it is suitable to use laminin as a coating agent (culture substrate) for the cell culture equipment, as the extracellular matrix. Since Vitronectin or Vitronectin+Col I is considered to be a collagen-based coating agent (culture substrate), it was considered that laminin or a laminin fragment (more preferably laminin 511E8 fragment) are suitable for inducing differentiation from a pluripotent stem cell to an epidermal keratinocyte.

### <Test Example 1-3: Examination of optimization of the period from seeding to initiation of induction>

In the pluripotent stem cell culture maintenance step of <Production Example 1> before the induction step of <Production Example 2>, the number of days from the seeding date to the initiation of induction was set to 3 days, 4 days, 5 days, and 6 days, and it was determined whether 100% confluence was achieved during the induction period (P=0) of <Production Example 2> as the criterion ∘ (A).

If the initiation of induction is the reference date and the previous day is -1 day before, etc., the "Days to initiation of indcuction" column in Table 1 will include -3 days, -4 days, -5 days, and -6 days from the top.

As shown in Table 1, it was confirmed that the shape and number of the cells were good by observation with an optical microscope when induction is performed using cells in which the number of days from the date of seeding to the initiation of induction is 3 to 6 days (more preferably 3 to 5 days) (see Figure 4).

### [Table 1]

**Table 1**

| Days from seeding to initiation of induction | Cell state (number) at the initiation of induction | Cell state (number and shape) during culture after initiation of induction | Total judgment |
|---|---|---|---|
| 3 days | Good | Cell state is good | ○(A) |
| 4 days | Slightly dead | Cell state is good | ○(A) |
| 5 days | Slightly dead | Cell state is good | ○(A) |
| 6 days | Slightly more deaths than for 5 days | Cell state is good | Δ(B) |

### <Test Example 1-4: Examination of the type of cell detaching agent during subculture operation>

Regarding the cell detaching agent used for subculture P=1 to divide into detached cell lines and non-detached cell lines in <Production Example 2>, after treating with the cell detaching agent in Table 2, the detachment state immediately after the treatment with the cell detaching agent and the state of cells after the treatment were investigated using an optical microscope. The cell detaching agents in Table 2 are TrypLE (trademark) select, actinase (produced by Godo Shusei Co., Ltd.), trypsin (trypsin/EDTA solution: produced by Kurabo Industries Ltd.), dispase (produced by Kaken Pharmaceutical Co., Ltd.), Accutase (produced by Innovative Cell Technologies, Inc.), and ReLeSR (produced by Veritas Corp.). Trypsin (EC.3.4.21.4) is a type of endopeptidase and serine protease, and is a type of digestive enzyme contained in pancreatic juice, which hydrates the peptide bond on the carboxy group side of basic amino acids (lysine, arginine).

Among the cell detaching agents used in Table 2, TrypLE (trademark) select enzyme was able to appropriately detach cells from the cell culture equipment immediately after treatment with the cell detaching agent, and further for 4 hours after treatment with the cell treatment agent, both the number and shape of the cells were good. In addition, trypsin was also able to appropriately detach cells from the cell culture equipment immediately after treatment with the cell detaching agent, and further for 4 hours after treatment with the cell treatment agent, both the number and shape of the cells were good. Although actinase and dispase were able to appropriately detach cells from the cell culture equipment, the number and shape of the cells afterward were somewhat unfavorable.

Furthermore, regarding the TrypLE (trademark) select enzyme, even when the pluripotent stem cell maintenance step period is 4 days, 5 days, and 6 days, it can detach cells almost or moderately, and furthermore, after 4 hours passed after treatment with the cell detaching agent, the state of the cells was good. In addition, regarding trypsin, even when the pluripotent stem cell maintenance step period was 5 days, it can detach cells almost or moderately, and furthermore, after 4 hours passed after treatment with the cell detaching agent, the state of the cells was good.

The present inventors believe that TrypLE (trademark) select enzyme is a substitute for porcine trypsin and may be classified as a trypsin-like protease.

Among these, the best enzymes were found to be trypsin-based enzymes, more specifically TrypLE (trademark) select enzyme and trypsin.

From these, the present inventors supposed that trypsin-based enzymes are preferred as the cell detaching agent, more specifically proteases and TrypLE (trademark) select enzymes are preferred, and more preferred are trypsin, trypsin-like proteases, and TrypLE (trademark) select enzyme, and still more preferred are trypsin and TrypLE (trademark) select enzyme.

### [Table 2]

**Table 2**

| Cell detaching agent | Enzyme property | Detached state immediately after treatment |
|---|---|---|
| TrypLE (trademark) select | Trypsin-like proteolytic enzyme | Detached appropriately *(*20-80% detached); Cells survive |
| Trypsin | Serine protease | Detached appropriately Cells survive |
| Actinase | Ca-dependent protease | Detached appropriately |
| Dispase | Metalloprotease | Detached appropriately |
| Accutase | Protease and collagenase activity (milder than trypsin) | Almost not detached |
| ReLeSR | Non-enzyme (enzyme free) | Almost not detached |

## Claims

1. A method for inducing differentiation from a pluripotent stem cell to an epidermal keratinocyte, using a non-detached cell after being treated with a cell detaching agent in an inducing step from the pluripotent stem cell,
wherein when applying the epidermal keratinocyte to a three-dimensional cultured skin model, a three-dimensional cultured skin model in which the horny cell layer is multi-layered is obtained.

2. The method for inducing differentiation from a pluripotent stem cell to an epidermal keratinocyte according to claim 1, wherein the induction is initiated 3 to 6 days after seeding the pluripotent stem cell.

3. The method for inducing differentiation from a pluripotent stem cell to an epidermal keratinocyte according to claim 1 or 2, wherein the cell detaching agent contains one or two selected from trypsin and trypsin-like protease.

4. The method for inducing differentiation from a pluripotent stem cell to an epidermal keratinocyte according to claim 1 or 2, wherein
the non-detached cell is a cell that has been obtained by treating the surface of the cell culture equipment with a culture substrate, then seeding and culturing the cell, before treating with cell detaching agent, and then treating with the cell detaching agent.

5. The method for inducing differentiation from a pluripotent stem cell to an epidermal keratinocyte according to claim 1 or 2, wherein
the non-detached cell is a cell that has been obtained by treating the surface of the cell culture equipment with a culture substrate, then seeding and culturing the cell, before treating with the cell detaching agent, and then treating with the cell detaching agent, and
the culture substrate is laminin or a fragment thereof.

6. The method for inducing differentiation from a pluripotent stem cell to an epidermal keratinocyte according to claim 1 or 2, wherein the non-detached cell is based on detached cells collected after culturing after initiation of the induction and performing detachment treatment with a cell detaching agent during the culturing.

7. The method for inducing differentiation from a pluripotent stem cell to an epidermal keratinocyte according to claim 1 or 2, wherein the non-detached cell is used for the next culture.

8. The method for inducing differentiation from a pluripotent stem cell to an epidermal keratinocyte according to claim 1 or 2, wherein the pluripotent stem cell is an artificial pluripotent stem cell (iPS cell).

9. The method for inducing differentiation from a pluripotent stem cell to an epidermal keratinocyte according to claim 1 or 2, wherein
the pluripotent stem cell is an artificial pluripotent stem cell, and
the artificial pluripotent stem cell is an artificial pluripotent stem cell derived from epidermal keratinocytes.

10. A method for producing an epidermal keratinocyte, comprising a step of inducing differentiation from a pluripotent stem cell to an epidermal keratinocyte, using a non-detached cell after being treated with a cell detaching agent.

11. A method for producing a three-dimensional cultured skin model using an epidermal keratinocyte obtained by the method for producing an epidermal keratinocyte according to claim 10.
